# EUROPÄISCHE PATENTSCHRIFT

(11) **EP 2 335 656 B1**
(45) Veröffentlichungstag und Bekanntmachung des Hinweises auf die Patenterteilung: **05.09.2012**
(21) Anmeldenummer: 10014938.4
(22) Anmeldetag: 06.02.2003
(51) Int. Cl.: A61F 2/44

(54) **Zwischenwirbelimplantat**
Implant between vertebrae
Implant intervertébral

(43) Veröffentlichungstag der Anmeldung: 22.06.2011
(62) Teilanmeldung aus: 03700311.8
(73) Patentinhaber: Synthes GmbH, 4436 Oberdorf (CH)
(72) Erfinder: Lechmann, Beat, 2544 Bettlach (CH); Burkard, Dominique, 5014 Gretzenbach (CH); Cain, Chris M. J., 5067 Norwood, S.A. (AU); Mathieu, Claude, 8001 Zürich (CH)
(74) Vertreter: Lusuardi, Werther

(56) Entgegenhaltungen:
- EP-A2- 0 974 319
- FR-A1- 2 747 034
- US-A1- 2002 016 595
- US-A1- 2002 169 508

## Beschreibung

Die Erfindung betrifft ein Zwischenwirbelimplantat, gemäss dem Oberbegriff des Patentanspruchs 1.

Aus der GB-A-2 207 607 ist ein Zwischenwirbelimplantat bekannt, welches eine hufeisenförmige Gestalt aufweist mit einer Mehrzahl von zylindrischen Löchern. Die Löcher sind innen glatt ausgebildet und weisen lediglich einen Anschlag für die Köpfe der darin einzuführenden Knochenschrauben auf. Die Nachteile dieser Anordnung bestehen darin, dass die darin eingeführten Befestigungsschrauben nur mit ihrem Schaft im Knochen verankert werden können, ohne dass eine rigide Verbindung mit dem hufeisenförmigen Zwischenwirbelimplantat resultiert. Sobald es zu einer Schwächung der Verankerung des Schraubenschaftes im Knochen kommt, wird das Zwischenwirbelimplantat beweglich gegenüber der Schraube und es besteht die Tendenz einer Migration der Knochenschrauben unter Gefährdung der Blutgefässe. Die Lockerung des Zwischenwirbelimplantates kann zudem zu einer Pseudoarthrose führen.

Aus der US-A 2000/0010511 MICHELSON ist ein Zwischenwirbelimplantat bekannt, welches an seiner Vorderfläche zwei Bohrungen mit einem Innengewinde aufweist, in welche Knochenschrauben mit einem Gewindekopf einführbar sind. Nachteilig bei diesem Implantat ist einerseits der Umstand, dass sich die Knochenschrauben wieder lockern können und gegen ein Herausdrehen oder Herausfallen nicht gesichert sind. Anderseits besteht der weitere Nachteil, dass die Knochenschrauben vollständig am Implantatkörper selbst befestigt sind und letzterer deshalb eine relativ grosse Belastung erfährt.

Schrauben, die am anterioren oder antero-lateralen Rand des Wirbelkörpers heraustreten, riskieren Hauptgefässe wie die Aorta, Vena cava sowie Versorgungsgefässe wie lumbale Arterien und Venen zu verletzen. Die Verletzung der Hauptgefässe hat die innere Verblutung innerhalb kürzester Zeit zur Folge. Das Lockern von Schrauben ist eher möglich, wenn sie nicht winkelstabil angebracht sind.

Hier will die Erfindung Abhilfe schaffen. Der Erfindung liegt das Problem zugrunde, ein Zwischenwirbelimplantat zu schaffen, welches mit Knochenfixationsmitteln eine dauerhaft rigide Verbindung eingehen kann, so dass auch bei einer Schwächung der Knochenstruktur keine Lockerung zwischen Zwischenwirbelimplantat und Knochenfixationsmitteln auftritt. Zudem soll über eine separat ausgebildete Frontplatte eine Zuggurtung für die Knochenfixationselemente erfolgen, so dass der Implantatkörper weniger Stress, d.h. überlagerte Spannungen, erfährt.

Die Erfindung löst die gestellte Aufgabe mit einem Zwischenwirbelimplantat, welches die Merkmale des Anspruchs 1 aufweist. Der Stand der Technik nach US-A-2002/0169508 ist im Oberbegriff gewürdigt.

Die durch die Erfindung erreichten Vorteile ergeben sich im wesentlichen durch die dauerhaft rigide, d.h. feste Verbindung zwischen dem Zwischenwirbelimplantat und den zu seiner Befestigung verwendeten longitudinalen Fixationselementen.

An der Vorderfläche des dreidimensionalen Körpers ist eine zur horizontalen Mittelebene des Zwischenwirbelimplantates vertikal angeordnete Frontplatte angebracht, durch welche die Bohrungen hindurchgehen und in welcher die longitudinalen Fixationselemente verankerbar sind. Dies hat gegenüber zweiteiligen Implantaten gemäss dem Stand der Technik, bei welchen in einem gesonderten Operationsschritt eine Frontplatte implantiert wird, den Vorteil, dass die Implantation des Zwischenwirbelimplantates einschrittig und damit einfacher und schneller durchführbar ist. Ein weiterer Vorteil liegt darin begründet, dass dadurch die Fixation des Zwischenwirbelimplantates möglichst frontal am Wirbelkörper erfolgt, d.h. dort wo in der Regel gutes Knochenmaterial vorhanden ist. Das Resultat ist eine anteriore Bewegungseinschränkung, ohne dass dadurch ein grösseres Risiko für die umliegenden Strukturen entsteht als dies mit einem Zwischenwirbelimplantat gemäss dem Stand der Technik der Fall ist. Die Last wird vom Zwischenwirbelimplantat unter Kompression immer noch aufgenommen und nicht durch die Frontplatte oder die Fixationsschrauben.

Bei einer besonderen Ausführungsform ist parallel zur Frontplatte eine Sicherungsplatte befestigbar, vorzugsweise mittels einer Schraubverbindung, eines Bajonettverschlusses oder eines Klickverschlusses. Die Sicherungsplatte weist zweckmässigerweise eine zentrale Bohrung auf, welche vorzugsweise mit einem Innengewinde versehen ist. Die Frontplatte weist zweckmässigerweise eine zentrale Bohrung zur Aufnahme eines Befestigungsmittels auf. Die Sicherungsplatte gestattet die simultane Sicherung sämtlicher Knochenfixationselemente.

Bei einer besonderen Ausführungsform ist mindestens eine der Bohrungen in der Frontplatte derart ausgebildet, dass ein darin aufgenommenes longitudinales Fixationselement in rigider Weise mit der Frontplatte verbindbar ist.

Zu einer rigiden Verbindung kann man beispielsweise dadurch gelangen, dass mindestens eine der Bohrungen ein Innengewinde aufweist. Eine entsprechende Knochenschraube mit einem Gewindekopf kann dann in rigider Weise mit dem Implantat verschraubt werden.

Eine Alternative dazu besteht darin, dass sich mindestens eine der Bohrungen gegen die Unterseite hin konusförmig verjüngt, so dass eine Knochenschraube mit korrespondierendem Konuskopf darin rigide verankert werden kann. Die konische Bohrung besitzt vorzugsweise einen Konuswinkel, der kleiner als der resultierende Reibungswinkel ist. Zweckmässigerweise beträgt die Konizität der konischen Bohrung 1 : 3,75 bis 1 : 20,00, vorzugsweise 1 : 5 bis 1 : 15.

Die Knochenfixationselemente können entweder einen glatten Kopf aufweisen, so dass keine rigide Verbindung mit dem Implantat erfolgt, oder aber einen Gewindekopf, Konuskopf oder Spreizkopf aufweisen, so dass eine rigide Verbindung mit dem Implantat erfolgt. In beiden Fällen werden aber die Knochenfixationselemente durch die Sicherungsplatte gegen ein späteres Herausdrehen, Herausstoßen oder Herausfallen gesichert.

Bei einer besonderen Ausführungsform ist die Frontplatte im dreidimensionalen Körper als Einsatz ausgebildet und vorzugsweise vertikal zur horizontalen Mittelebene verschieblich angeordnet, so dass sie sich gegenüber dem dreidimensionalen Körper in der Vertikalen verschieben kann. Dadurch erreicht man ein "stress shielding" (Schutz, bzw. Neutralisierung von mechanischen Spannungen), welches eine graduelle Anpassung der Endplatten an das Zwischenwirbelimplantat während des Heilungsprozesses erlaubt.

Bei einer weiteren Ausführungsform ist die Frontplatte aus einem anderen Material gefertigt als der dreidimensionale Körper, vorzugsweise aus einem metallischen Werkstoff. Als metallische Werkstoffe eigenen sich insbesondere Titan oder Titanlegierungen. Die komplette Zuggurtungsanordnung (Front platte und Schrauben) kann auch aus Implantatstahl oder hochlegierten metallische Werkstoffen wie CoCrMo der CoCrMoC gefertigt sein. Der Vorteil des Titans liegt in der Gewebeverträglichkeit und dem guten Knocheneinwachsverhalten. der Vorteil des hochlegierten metallischen Werkstoffs liegt in ihren hohen Festigkeitswerten, die filigrane Konstruktionen erlauben.

Zweckmässigerweise sind die Oberseite und/oder die Unterseite des Zwischenwirbelimplantats nicht planar, sondern vorzugsweise konvex ausgebildet. Damit kann eine bessere Anpassung an die Endplatten der benachbarten Wirbelkörper erreicht werden. Bei einer weiteren Ausführungsform sind die Seitenflächen des Zwischenwirbelimplantats im Wesentlichen alle konvex ausgebildet.

Zweckmässigerweise durchbohren die Bohrungen die linke und die rechte Seitenfläche des Zwischenwirbelimplantats nicht.

Bei einer weiteren bevorzugten Ausführungsform verlaufen mindestens zwei der Bohrungen parallel. Dadurch wird die Einführbarkeit des Zwischenwirbelimplantats bei der Implantation erleichtert.

Bei einer anderen bevorzugten Ausführungsform verlaufen mindestens zwei der Bohrungen von der Vorderseite aus betrachtet divergent. Dadurch gelangt man mit den Knochenschrauben in einen Bereich des Wirbelkörpers, der im Vergleich zu dessen Zentrum eine bessere Knochenqualität aufweist.

Bei einer besonderen Ausführungsform schliessen die Achsen der Bohrungen zur horizontalen Mittelebene einen Winkel beta im Bereich von 20° bis 60°, vorzugsweise von 36° bis 48° ein. Die Achsen der Bohrungen schliessen zur vertikalen Mittelebene zweckmässigerweise einen Winkel alpha im Bereich von 10° bis 45°, vorzugsweise von 27° bis 33° ein. Damit wird ein besserer Zugang bei der Einbringung der Schrauben erreicht.

Bei einer weiteren Ausführungsform wird die horizontale Mittelebene von den Bohrungen nicht durchstossen.

Bei einer besonderen Ausführungsform ist die Oberseite und Unterseite des Körpers mit einer Strukturierung, vorzugsweise in Form von Zähnen versehen.

Das Zwischenwirbelimplantat kann als Hohlkörper ausgebildet sein, dessen Mantelflächen vorzugsweise mit Perforationen versehen sind.

Je nach den Umständen können zwei, drei, vier oder auch mehr longitudinale Fixationselemente in rigider Weise mit dem Zwischenwirbelimplantat verbunden werden, zweckmässigerweise sollte mindestens ein Fixationselement die Oberseite und mindestens ein Fixationselement die Unterseite des Zwischenwirbelimplantat durchstossen.

Vorzugsweise werden longitudinale Fixationselemente in Form von Knochenschrauben mit einem Kopf und einem Schaft verwendet, wobei der Kopf vorzugsweise mit einem Aussengewinde versehen ist, welches mit dem Innengewinde der Bohrung des Zwischenwirbelimplantats korrespondiert. Bei einer zweiten möglichen rigiden Verbindungsart kann vorzugsweise eine Knochenschraube verwendet werden, bei welcher sich der Kopf gegen den Schaft hin konisch verjüngt, wobei die Konizität des Kopfes der Konizität der Bohrung des Zwischenwirbelimplantates entspricht.

Bei einer weiteren Ausführungsform durchstossen mindestens zwei longitudinale Fixationselemente die Oberseite und mindestens zwei longitudinale Fixationselement die Unterseite. Damit erhält das Zwischenwirbelimplantat eine optimale Verankerung in den benachbarten Wirbelkörpern.

Die als Knochenschrauben ausgebildeten, longitudinalen Fixationselemente weisen vorzugsweise ein selbstbohrendes und selbstschneidendes Aussengewinde auf. Die longitudinalen Fixationselemente können auch als gewindelose Zylinderstifte ausgebildet sein, welche mit einer Bohrerspitze, vorzugsweise in Form eines Trokars versehen sind.

Eine weitere Variante besteht darin, dass die longitudinalen Fixationselemente als Spiralfedern ausgebildet sind und schliesslich können die longitudinalen Fixationselemente auch als ein- oder mehrflügelige Spiralklingen ausgebildet sein.

Das Zwischenwirbelimplantat kann aus irgendeinem körperverträglichen Material gefertigt werden, zweckmässigerweise besteht jedoch der Körper aus einem körperverträglichen Kunststoff, vorzugsweise einem unverstärkten Kunststoff. Der Vorteil gegenüber den in der Implantologie bereits bekannten faserverstärkten Kunststoffen besteht darin, dass keine Verstärkungsfasern freigelegt werden, was klinisch nachteilig ist. In einen solchen aus unverstärktem Kunststoff bestehenden Körper, können zweckmässigerweise Knochenschrauben verwendet werden, deren Aussengewinde einen Lastflankenwinkel im Bereich von 11° bis 14°, vorzugsweise von 12° bis 13° aufweist. Die vergleichsweise geringe Neigung der Lastflanke bewirkt eine hohe Klemmkraft, wodurch die Radialdehnung und die Rissgefahr im Kunststoff reduziert werden. Zweckmässigerweise weist das Aussengewinde der Knochenschrauben einen Steigungswinkel im Bereich von 6° bis 10°, vorzugsweise von 7° bis 9° auf. Dieser spezielle Steigungswinkel erzeugt eine Selbsthemmung im Gewinde und sichert damit die Knochenschraube gegen ein selbständiges Lösen.

Um die Verankerung der Knochenschraube im Kunststoffkörper zu verbessern, kann die Bohrung eine Metallhülse mit Innengewinde sein. Das Zwischenwirbelimplantat kann auch nur teilweise aus einem röntgenstrahlendurchlässigen Kunststoff und - im Bereich der Bohrungen - aus Metall, z.B. aus Titan oder Titanlegierung bestehen. Damit wird insgesamt eine bessere Führung und Verankerung der Knochenschrauben im Zwischenwirbelimplantat erreicht.

Bei einer weiteren bevorzugten Ausführungsform können die Bohrungen eine glatte Innenwand aufweisen, in welche der Gewindekopf eines metallischen, longitudinalen Fixationselementes eingeschnitten oder eingeformt werden kann.

Die Erfindung und Weiterbildungen der Erfindung werden im Folgenden anhand der teilweise schematischen Darstellung eines Ausführungsbeispiels noch näher erläutert.

Es zeigen:
Fig. 1 eine Explosionszeichnung des erfindungsgemässen Zwischenwirbelimplantates mit einer Sicherungsplatte;
Fig. 2 ein longitudinales Knochenfixationsmittel in Form einer Schraube;
Fig. 3 eine Vorderansicht des zusammengesetzten Zwischenwirbelimplantates nach Fig. 1;
Fig. 4 eine Seitenansicht des zusammengesetzten Zwischenwirbelimplantates nach Fig. 1;
Fig. 5 eine dreidimensionale Detailansicht des Körpers des Zwischenwirbelimplantates, welche die Verbindungselemente zur Frontplatte nach Fig. 6 zeigt;
Fig. 6 eine dreidimensionale Detailansicht der Frontplatte des Zwischenwirbelimplantates, welche die Verbindungselemente zum Körper nach Fig. 5 zeigt; und
Fig. 7 ein komplett montiertes Zwischenwirbelimplantat mit Frontplatte gemäss der Erfindung und mit einer Sicherungsplatte.

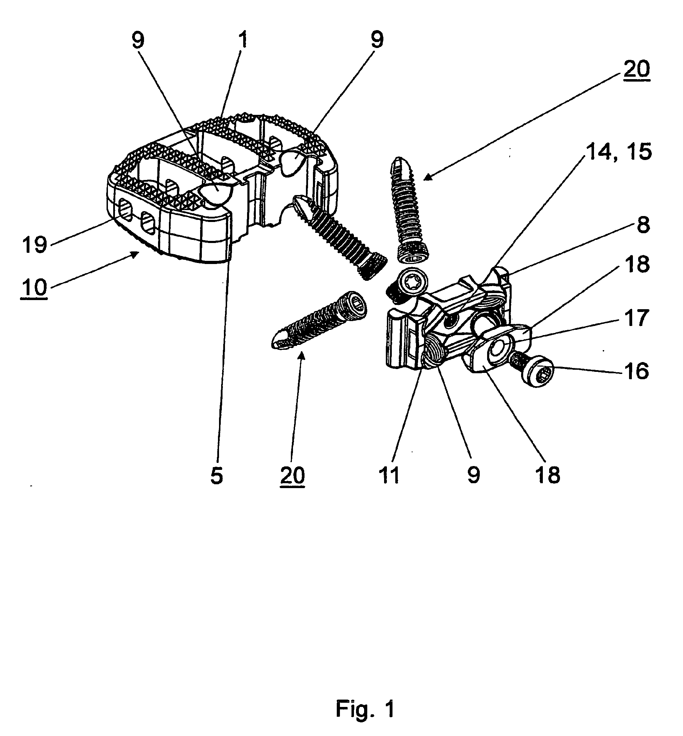

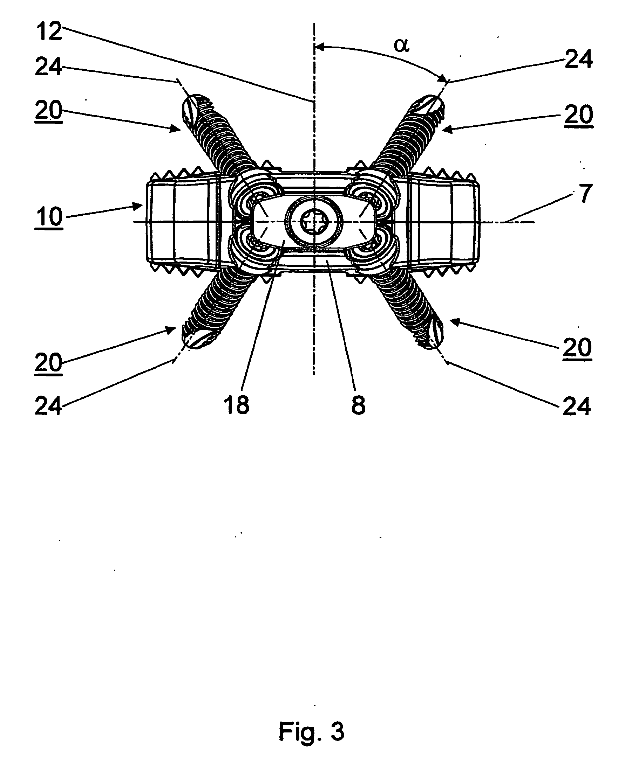

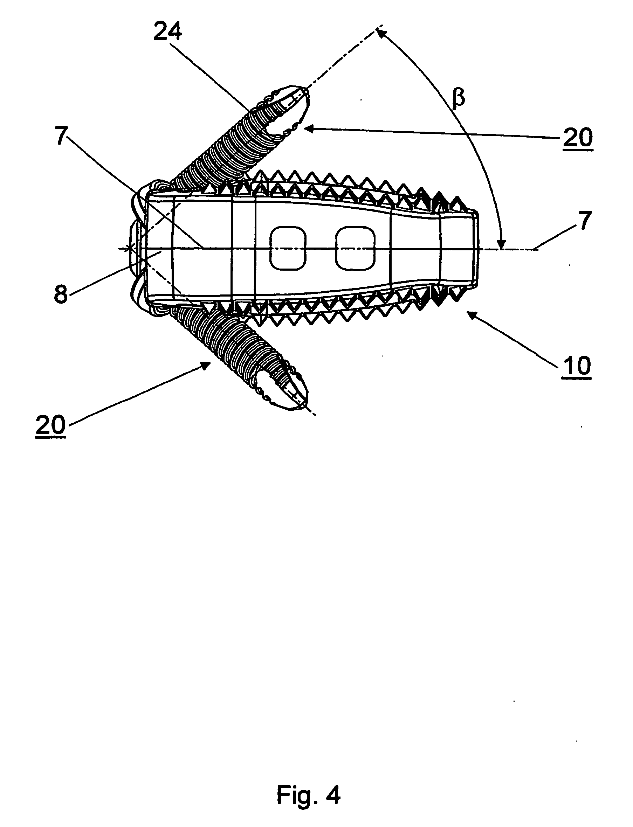

Das in den Fig. 1 - 7 dargestellte Zwischenwirbelimplantat umfasst einen dreidimensionalen Körpers 10 in Form eines Käfigs mit einer Oberseite 1 und einer Unterseite 2, welche zur Anlage an die Endplatten zweier benachbarter Wirbelkörper geeignet sind, einer linken Seitenfläche 3 und einer rechten Seitenfläche 4, einer Vorderfläche 5 und einer Hinterfläche 6, einer zwischen der Oberseite 1 und Unterseite 2 liegenden, horizontalen Mittelebene 7, einer von der Vorderfläche 5 zur Hinterfläche 6 verlaufenden vertikalen Mittelebene 12, vier den Körper 10 durchdringende Bohrungen 9, die zur Aufnahme von longitudinalen Fixationselementen 20 geeignet sind. Der dreidimensionale Körper 10 weist an seiner Vorderfläche 5 eine Frontplatte 8 auf, durch welche die Bohrungen 9 hindurchgehen und in welcher die longitudinalen Fixationselemente 20 verankerbar sind. Keine der vier Bohrungen (9) durchbohrt die Vorderfläche 5 des Körpers 10 vollständig. Der Körper 10 ist als Hohlkörper ausgebildet, dessen Mantelflächen mit Perforationen 19 versehen sind.

Das Zwischenwirbelimplantat weist im weiteren eine Sicherungsplatte 18 auf, welche mittels einer Schraubverbindung parallel zur Frontplatte 8 an der Frontplatte 8 derart befestigbar ist, dass die Bohrungen 9 teilweise von der Sicherungsplatte 18 abdeckbar sind. Die Sicherungsplatte 18 weist zu diesem Zweck eine zentrale Bohrung 17 auf. Dazu entsprechend weist die Frontplatte 8 eine zentrale Bohrung 15 mit Innengewinde 14 zur Aufnahme eines Befestigungsmittels 16 in Form einer Schraube auf. Die Sicherungsplatte 18 kann auch weggelassen werden.

Die vier Bohrungen 9 in der Frontplatte 8 weisen ein Innengewinde 11 auf, so dass die darin aufgenommenen longitudinalen Fixationselemente 20 in Form von Schrauben in rigider Weise mit der Frontplatte 8 verbindbar sind.

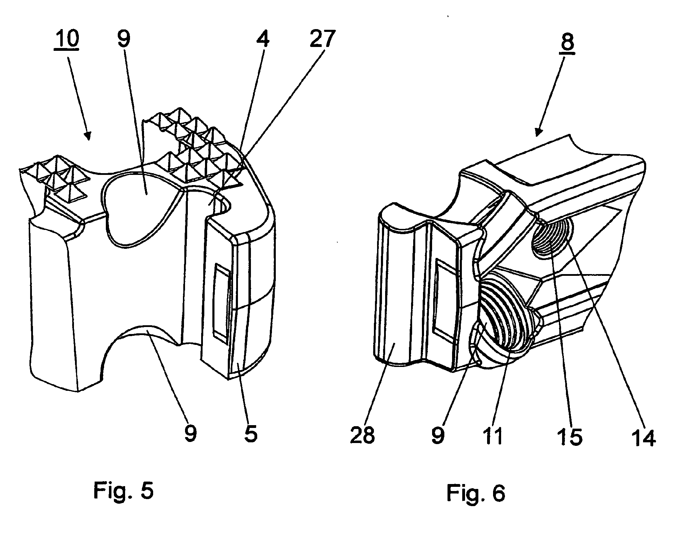

Die Frontplatte 8 besteht aus Titan und der dreidimensionale Körper 10 aus einem röntgenstrahlendurchlässigen, unverstärkten Kunststoff. Die Frontplatte 8 ist wie in den Fig. 5/6 dargestellt als Einsatz für den Körper 10 ausgebildet und vertikal zur horizontalen Mittelebene 7 verschieblich angeordnet. Der Körper 10 weist zu diesem Zweck an den Übergängen der linken Seitenfläche 3, bzw. der rechten Seitenfläche 4 (Fig. 5) zur Vorderfläche 5 ein parallel zur vertikalen Mittelebene 12 verlaufende, halbkreiszylinderförmige Nut 27 auf. Entsprechen weist die Frontplatte 8 rechts und links (Fig. 6) eine gleich verlaufende und gleich dimensionierte, halbkreiszylinderförmige Schiene 28 auf. Dadurch lässt sich die Frontplatte - bei der Herstellung des Zwischenwirbelimplantates - mit ihren beiden lateralen Schienen 28 leicht in die entsprechenden Nuten 27 des Körpers 10 einschieben und positionieren.

Die Seitenflächen 1, 2, 3, 4, 5 und 6 des Körpers 10 sind alle konvex ausgebildet.

Die Bohrungen 9 durchbohren weder die linke Seitenfläche 3 noch die rechte Seitenfläche 4; auch die Vorderfläche 5 wird von den Bohrungen 9 nicht vollständig durchbohrt.

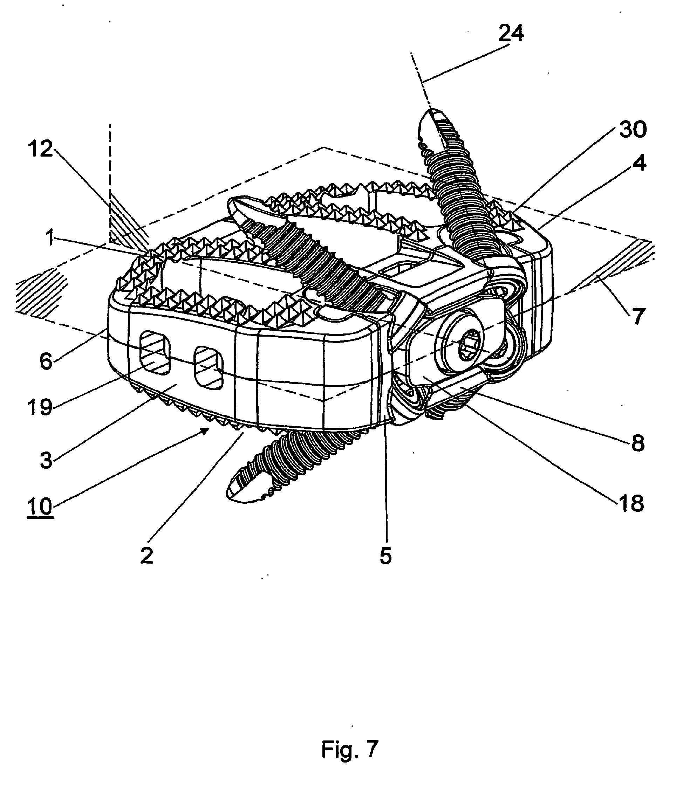

Die vier Bohrungen 9 verlaufen von der Vorderfläche 5 aus betrachtet alle divergent (Fig. 7).

Die Achsen 24 der Bohrungen 9 schliessen zur horizontalen Mittelebene 7 einen Winkel beta von 42° ein und zur vertikalen Mittelebene 12 einen Winkel alpha von 30° ein.

Die Bohrungen 9 durchstossen die horizontale Mittelebene 7 nicht, lediglich die Achsen 24 der darin eingeführten longitudinalen Fixationselementen 20 schneiden die horizontale Mittelebene 7 des Körpers 10.

Wie in Fig. 7 dargestellt ist die Oberseite 1 und Unterseite 2 des Körpers 10 mit einer Strukturierung in Form von Zähnen 30 versehen.

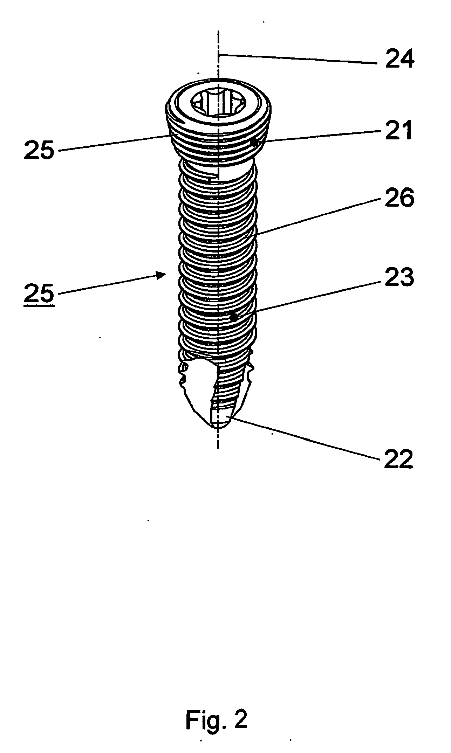

Die longitudinalen Fixationselemente 20 sind als Knochenschrauben ausgebildet. Wie in Fig. 2 dargestellt weisen die in die Bohrungen 9 eingeführten longitudinalen Fixationselemente 20 einen Kopf 21, eine Spitze 22, einen Schaft 23 und eine Achse 24 auf. Der Kopf 21 ist mit einem Aussengewinde 25 versehen, welches mit dem Innengewinde 11 der Bohrung 9 korrespondiert, so dass die Köpfe 21 in den Bohrungen 9 in rigider Weise verankerbar sind. Der Schaft 23 ist mit einem Gewinde 26 versehen ist, welches selbstbohrend und selbstschneidend ist. Der Lastflankenwinkel des Gewindes 26 beträgt 12,5° und der Steigungswinkel 8°.

Durch das Befestigen der Sicherungsplatte 18 an der Frontplatte 8 werden die Köpfe 21 der longitudinalen Fixationselemente 20 von der Sicherungsplatte 18 kontaktiert, so dass sie gegen ein Herausstossen oder Herausdrehen gesichert sind.

Wie in Fig. 7 dargestellt durchstossen zwei longitudinale Fixationselemente 20 die Oberseite 1 und zwei longitudinale Fixationselemente 20 die Unterseite 2 des Körpers 10.

Zur weiteren Erläuterung der Erfindung folgt ein kurzer Operationsbeschrieb:
a) Das Zwischenwirbelimplantat in Form eines dreidimensionalen Körpers (10) wird mittels eines geeigneten Instruments zwischen zwei benachbarte Wirbelkörper eingebracht;
b) vier longitudinale Fixationselemente 20 in Form von Knochenschrauben werden mittels eines geeigneten Zielgerätes durch die Bohrungen 9 der Frontplatte 8 in die Wirbelkörper eingeschraubt;
c) die Sicherungsplatte 18 wird mittels des Befestigungsmittels 16 in Form einer Schraube über den Köpfen 21 der longitudinalen Fixationselemente 20 an der Frontplatte befestigt, so dass die Köpfe 21 der longitudinalen Fixationselemente 20 - und damit die Schrauben selbst - zwischen der Frontplatte 8 und der Sicherungsplatte 18 gefangen und gegen eine relative Verschiebung zum Körper 10 (z.B. durch Herausfallen oder Herausdrehen) gesichert sind. Das Befestigungsmittel 16 in Form einer Schraube ist vorzugsweise mit einem Gewinde versehen, welches sich durch eine grosse Selbsthemmung auszeichnet.

## Patentansprüche

1. Zwischenwirbelimplantat, das einen dreidimensionalen Körper (10) umfasst mit
A) einer Oberseite (1) und einer Unterseite (2), welche zur Anlage an die Endplatten zweier benachbarter Wirbelkörper geeignet sind;
B) einer linken Seitenfläche (3) und einer rechten Seitenfläche (4);
C) einer Vorderfläche (5) und einer Hinterfläche (6);
D) einer zwischen der Oberseite (1) und Unterseite (2) liegenden, horizontalen Mittelebene (7);
E) einer von der Vorderfläche (5) zur Hinterfläche (6) verlaufenden vertikalen Mittelebene (12);
F) der dreidimensionale Körper (10) an seiner Vorderfläche (5) eine Frontplatte (8) aufweist, durch welche eine Mehrzahl von Bohrungen (9) hindurchgehen und in welcher longitudinale Fixationselemente (20) verankerbar sind; und
G) die Mehrzahl von Bohrungen (9) den Körper (10) durchdringen und zur Aufnahme der longitudinalen Fixationselementen (20) geeignet sind,
**dadurch gekennzeichnet, dass**
H) die Bohrungen (9) nicht vollständig durch die Vorderfläche (5) des Körpers (10) hindurchgehen.

2. Zwischenwirbelimplantat nach Anspruch 1, **dadurch gekennzeichnet, dass** keine der Bohrungen (9) den Körper (10) vollständig durchbohren.

3. Zwischenwirbelimplantat nach Anspruch 1 oder 2, **dadurch gekennzeichnet, dass** es eine Sicherungsplatte (18) aufweist, welche im Wesentlichen parallel zur Frontplatte (8) am Körper (10) oder an der Frontplatte (8) derart befestigbar ist, dass die Bohrungen (9) mindestens teilweise von der Sicherungsplatte (18) abdeckbar sind.

4. Zwischenwirbelimplantat nach einem der Ansprüche 1 bis 3, **dadurch gekennzeichnet, dass** mindestens eine der Bohrungen (9) in der Frontplatte (8) derart ausgebildet ist, dass ein darin aufgenommenes longitudinales Fixationselement (20) in rigider Weise mit der Frontplatte (8) verbindbar ist.

5. Zwischenwirbelimplantat nach Anspruch 4, **dadurch gekennzeichnet, dass** mindestens eine der Bohrungen (9) ein Innengewinde (11) aufweist.

6. Zwischenwirbelimplantat nach einem der Ansprüche 1 bis 5, **dadurch gekennzeichnet, dass** sich mindestens eine Bohrung (9) gegen die Unterseite (2) hin konusförmig verjüngt.

7. Zwischenwirbelimplantat nach einem der Ansprüche 1 bis 6, **dadurch gekennzeichnet, dass** die Frontplatte (8) als Einsatz ausgebildet ist und vorzugsweise vertikal zur horizontalen Mittelebene (7) angeordnet ist.

8. Zwischenwirbelimplantat nach einem der Ansprüche 1 bis 7, **dadurch gekennzeichnet, dass** die Frontplatte (8) aus einem anderen Material gefertigt ist als der dreidimensionale Körper (10) und vorzugsweise metallisch ist.

9. Zwischenwirbelimplantat nach einem der Ansprüche 1 bis 8, **dadurch gekennzeichnet, dass** die Bohrungen (9) die linke Seitenfläche (3) und die rechte Seitenfläche (4) nicht durchbohren.

10. Zwischenwirbelimplantat nach einem der Ansprüche 1 bis 9, **dadurch gekennzeichnet, dass** mindestens zwei der Bohrungen (9) von der Vorderseite (1) aus betrachtet divergent verlaufen.

11. Zwischenwirbelimplantat nach einem der Ansprüche 1 bis 10, **dadurch gekennzeichnet, dass** die Achsen (24) der Bohrungen (9) zur horizontalen Mittelebene (7) einen Winkel beta im Bereich von 20° bis 60°, vorzugsweise von 36° bis 48° einschliessen.

12. Zwischenwirbelimplantat nach einem der Ansprüche 1 bis 11, **dadurch gekennzeichnet, dass** die Achsen (24) der Bohrungen (9) zur vertikalen Mittelebene (12) einen Winkel alpha im Bereich von 10° bis 45°, vorzugsweise von 27° bis 33° einschliessen.

13. Zwischenwirbelimplantat nach einem der Ansprüche 1 bis 12, **dadurch gekennzeichnet, dass** es teilweise aus einem röntgenstrahlendurchlässigen Material, vorzugsweise einem Kunststoff, und im Bereich der Bohrungen (9) aus einem Metall, vorzugsweise auf der Basis von Titan, besteht.

14. Zwischenwirbelimplantat nach einem der Ansprüche 1 bis 13, **dadurch gekennzeichnet, dass** dessen Oberseite (1) und Unterseite (2) mit einer Strukturierung, vorzugsweise in Form von Zähnen (30), versehen ist.

15. Zwischenwirbelimplantat nach einem der Ansprüche 1 bis 14, mit mindestens zwei in die Bohrungen (9) einführbaren longitudinalen Fixationselementen (20),
**dadurch gekennzeichnet, dass**
die in die Bohrungen (9) eingeführten longitudinalen Fixationselemente (20) einen Kopf (21), eine Spitze (22), einen Schaft (23) und eine Achse (24) aufweisen und dass die Köpfe (21) in den Bohrungen (9) verankerbar sind.

## Claims

1. Intervertebral implant, comprising a three-dimensional body (10) with
A) a top side (1) and an underside (2), suitable for abutting the end plates of two neighbouring vertebral bodies;
B) a left side surface (3) and a right side surface (4);
C) a front surface (5) and a rear surface (6);
D) a horizontal central plane (7) positioned between the top side (1) and underside (2);
E) a vertical central plane (12) running from the front surface (5) to the rear surface (6);
F) the three-dimensional body (10) is provided at its front surface (5) with a front plate (8), through which a plurality of bore holes (9) extend and in which longitudinal fixation elements (20) can be anchored;
**characterized in that**
G) the plurality of bore holes (9) penetrate through the body (10) and are suitable for receiving the longitudinal fixation elements (20); and
H) the bore holes (9) are not in its entirety penetrating the front surface (5) of the body (10).

2. Intervertebral implant according to claim 1, **characterized in that** none of the bore holes (9) completely penetrates through the body (10).

3. Intervertebral implant according to claim 1 or 2, **characterized in that** it comprises a securing plate (18) that can be secured essentially parallel to the front plate (8) on the body (10) or on the front plate (8) in such a way that the bore holes (9) can be covered at least partially by the securing plate (18).

4. Intervertebral implant according to one of the claims 1 to 3, **characterized in that** at least one of the bore holes (9) in the front plate (8) is configured in such a way that a longitudinal fixation element (20) received therein is connectable to the front plate (8) in a rigid manner.

5. Intervertebral implant according to claim 4, **characterized in that** at least one of the bore holes (9) is provided with an internal thread (11).

6. Intervertebral implant according to one of the claims 1 to 5, **characterized in that** at least one of the bore holes (9) tapers conically towards the underside (2).

7. Intervertebral implant according to one of the claims 1 to 6, **characterized in that** the front plate (8) is configured as an insert and is preferably arranged vertically to the horizontal central plane (7).

8. Intervertebral implant according to one of the claims 1 to 7, **characterized in that** the front plate (8) is manufactured from a material that is different to that used for the three-dimensional body (10) and that is preferably a metal.

9. Intervertebral implant according to one of the claims 1 to 8, **characterized in that** the bore holes (9) do not penetrate the left side surface (3) and the right side surface (4).

10. Intervertebral implant according to one of the claims 1 to 9, **characterized in that** at least two of the bore holes (9) run in a divergent direction when viewed from the front side (1).

11. Intervertebral implant according to one of the claims 1 to 10, **characterized in that** the axes (24) of the bore holes (9) in relation to the horizontal central plane (7) form an angle beta in the range 20° to 60°, preferably 36° to 48°.

12. Intervertebral implant according to one of the claims 1 to 11, **characterized in that** the axes (24) of the bore holes (9) in relation to the vertical central plane (12) form an angle alpha in the range 10° to 45°, preferably 27° to 33°.

13. Intervertebral implant according to one of the claims 1 to 12, **characterized in that** it consists partially of a material permeable to x-rays, preferably a plastic, and in the area of the bore holes (9) consists of a metal, preferably a metal based on titanium.

14. Intervertebral implant according to one of the claims 1 to 13, **characterized in that** the top side (1) and underside (2) are provided with a structuring, preferably in the form of teeth (30).

15. Intervertebral implant according to one of the claims 1 to 14, with at least two longitudinal fixation elements (20) that can be inserted into the bore holes (9), **characterized in that**
the longitudinal fixation elements (20) inserted into the bore holes (9) are provided with a head (21), a tip (22), a shaft (23) and an axis (24) and that the heads (21) can be anchored in the bore holes (9).

## Revendications

1. Implant intervertébral, qui comprend un corps (10) tridimensionnel avec
A) un côté supérieur (1) et un côté inférieur (2) qui sont appropriés pour l'appui sur les plaques d'extrémité de deux corps vertébraux voisins ;
B) une surface latérale gauche (3) et une surface latérale droite (4) ;
C) une surface avant (5) et une surface arrière (6) ;
D) un plan médian (7) horizontal situé entre le côté supérieur (1) et le côté inférieur (2);
E) un plan médian (12) vertical courant de la surface avant (5) à la surface arrière (6) ;
F) le corps (10) tridimensionnel présente sur sa surface avant (5) une plaque frontale (8) traversée par une multiplicité d'alésages (9) et dans laquelle des éléments de fixation (20) longitudinaux peuvent être ancrés ; et
G) la multiplicité d'alésages (9) pénètrent dans le corps (10) et sont appropriés pour recevoir les éléments de fixation (20) longitudinaux,
**caractérisé en ce que**
H) les alésages (9) ne traversent pas complètement la surface avant (5) du corps (10).

2. Implant intervertébral selon la revendication 1, **caractérisé en ce qu'**aucun des alésages (9) ne perce complètement le corps (10).

3. Implant intervertébral selon la revendication 1 ou la revendication 2, **caractérisé en ce qu'**il présente une plaque de blocage (18) qui peut être fixée essentiellement parallèlement à la plaque frontale (8) sur le corps (10) ou sur la plaque frontale (8) de sorte que les alésages (9) peuvent être recouverts au moins partiellement par la plaque de blocage (18).

4. Implant intervertébral selon une des revendications 1 à 3, **caractérisé en ce qu'**au moins un des alésages (9) dans la plaque frontale (8) est constitué de sorte qu'un élément de fixation (20) longitudinal qui y est reçu peut être raccordé de façon rigide à la plaque frontale (8).

5. Implant intervertébral selon la revendication 4, **caractérisé en ce qu'**au moins un des alésages (9) présente un filet intérieur (11).

6. Implant intervertébral selon une des revendications 1 à 5, **caractérisé en ce qu'**au moins un alésage (9) se rétrécit en forme de cône en direction du côté inférieur (2).

7. Implant intervertébral selon une des revendications 1 à 6, **caractérisé en ce que** la plaque frontale (8) est constituée en tant qu'insert et est disposée de préférence verticalement par rapport au plan médian (7) horizontal.

8. Implant intervertébral selon une des revendications 1 à 7, **caractérisé en ce que** la plaque frontale (8) est fabriquée dans un autre matériau que le corps (10) tridimensionnel et est de préférence métallique.

9. Implant intervertébral selon une des revendications 1 à 8, **caractérisé en ce que** les alésages (9) ne percent pas la surface latérale gauche (3) et la surface latérale droite (4).

10. Implant intervertébral selon une des revendications 1 à 9, **caractérisé en ce qu'**au moins deux des alésages (9) divergent quand ils sont vus à partir du côté supérieur (1).

11. Implant intervertébral selon une des revendications 1 à 10, **caractérisé en ce que** les axes (24) des alésages (9) forment, par rapport au plan médian (7) horizontal, un angle beta dans la plage de 20° à 60°, de préférence de 36° à 48°.

12. Implant intervertébral selon une des revendications 1 à 11, **caractérisé en ce que** les axes (24) des alésages (9) forment, par rapport au plan médian (12) vertical, un angle alpha dans la plage de 10° à 45°, de préférence de 27° à 33°.

13. Implant intervertébral selon une des revendications 1 à 12, **caractérisé en ce qu'**il est constitué d'un matériau transparent aux rayons X, de préférence une matière plastique et, dans la zone des alésages (9), d'un métal, de préférence à base de titane.

14. Implant intervertébral selon une des revendications 1 à 13, **caractérisé en ce que** son côté supérieur (1) et son côté inférieur (2) sont munis d'une structuration, de préférence en forme de dents (30).

15. Implant intervertébral selon une des revendications 1 à 14, avec au moins deux éléments de fixation (20) longitudinaux pouvant être introduits dans les alésages (9),
**caractérisé en ce que**
les éléments de fixation (20) longitudinaux introduits dans les alésages (9) présentent une tête (21), une pointe (22), une tige (23) et un axe (24), et **en ce que** les têtes (21) peuvent être ancrées dans les alésages (9).
